(19) Europäisches Patentamt
European Patent Office
Office européen des brevets

(11) Veröffentlichungsnummer: **0 561 250 B1**

(12) **EUROPÄISCHE PATENTSCHRIFT**

(45) Veröffentlichungstag der Patentschrift: **08.02.95**

(51) Int. Cl.[6]: **C07D 271/08**, A61K 31/41, C07C 22/04, C07C 25/02

(21) Anmeldenummer: **93103668.5**

(22) Anmeldetag: **08.03.93**

Die Akte enthält technische Angaben, die nach dem Eingang der Anmeldung eingereicht wurden und die nicht in dieser Patentschrift enthalten sind.

(54) **Furazanylharnstoffe.**

(30) Priorität: **19.03.92 DE 4208873**

(43) Veröffentlichungstag der Anmeldung:
**22.09.93 Patentblatt 93/38**

(45) Bekanntmachung des Hinweises auf die Patenterteilung:
**08.02.95 Patentblatt 95/06**

(84) Benannte Vertragsstaaten:
**BE CH DE ES FR GB IT LI NL**

(56) Entgegenhaltungen:
**EP-A- 0 132 680**
**EP-A- 0 156 198**
**EP-A- 0 189 043**
**EP-A- 0 253 175**
**EP-A- 0 300 968**
**EP-A- 0 316 734**

(73) Patentinhaber: **BAYER AG**
**D-51368 Leverkusen (DE)**

(72) Erfinder: **Sirrenberg, Wilhelm, Dr.**
**Wuppertaler Strasse 21**
**W-4322 Sprockhövel (DE)**
Erfinder: **Lantzsch, Reinhard, Dr.**
**Am Buschhäuschen 51**
**W-5600 Wuppertall (DE)**
Erfinder: **Marhold, Albrecht, Dr.**
**Carl-Duisberg-Strasse 329**
**W-5090 Leverkusen 1 (DE)**
Erfinder: **Wachendorff-Neumann, Ulrike, Dr.**
**Krischerstrasse 81**
**W-4019 Monheim (DE)**
Erfinder: **Elbert, Alfred, Dr.**
**Altenberger Strasse 23**
**W-5093 Burscheid (DE)**

EP 0 561 250 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Furazanylharnstoffe, ein Verfahren zu ihrer Herstellung und ihre Verwendung als Schädlingsbekämpfungsmittel, insbesondere als Akarizide.

Es ist bereits bekannt, daß bestimmte Furazanylharnstoffe, wie z.B. 1-(4-(4-Chlor-phenyl)-1,2,5-oxadiazol-3-yl)-3-(4-trifluormethoxy-phenyl)-harnstoff und 1-(4-(4-Chlor-phenyl)-1,2,5-oxadiazol-3-yl)-3-(2-chlor-4-(2,6-dichlor-4-trifluormethyl-phenoxy)-phenyl)-harnstoff, akarizide Eigenschaften aufweisen (vgl. EP-A 0 132 680 oder US-Pat. Nr. 4 699 916 sowie EP-A-0 316 734 oder US-Pat. 4 886 823). Weitere Furazanylharnstoffe mit akarziden Eigenschaften werden beschrieben in EP-A- 0 156 198, EP-A 0 189 043 und EP-A- 0 253 175. Aus der EP-A- 0 300 968 sind bestimmte Benzoylphenylharnstoffe als Schädlingsbekämpfungsmittel bekannt.

Es wurden nun die neuen Furazanylharnstoffe der allgemeinen Formel (I)

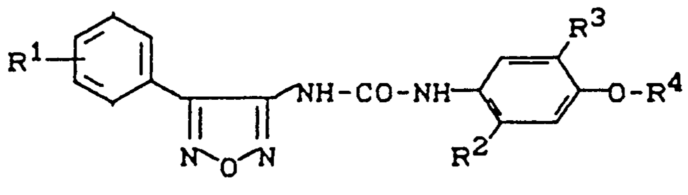

(I)

in welcher

- $R^1$ für Wasserstoff oder Fluor steht,
- $R^2$ für Wasserstoff, Fluor oder Chlor steht,
- $R^3$ für Wasserstoff, Fluor oder Chlor steht und
- $R^4$ für Phenyl steht, welches durch Trifluormethyl und zusätzlich einfach bis vierfach durch Fluor und/oder Chlor substituiert ist, und,
  für den Fall, daß $R^1$ für Fluor steht und/oder $R^2$ und/oder $R^3$ für Fluor oder Chlor stehen, $R^4$ darüber hinaus auch für Difluormethyl, Trifluormethyl, Chlordifluormethyl, Trifluorethyl, Tetrafluorethyl, Chlortrifluorethyl, Hexafluorpropyl, Hexafluorpropenyl oder Trifluormethylphenyl steht,

gefunden.

Weiter wurde gefunden, daß man die neuen Furazanylharnstoffe der allgemeinen Formel (I) erhält, wenn man Furazanylisocyanate der allgemeinen Formel (II)

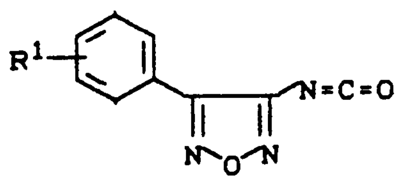

(II)

in welcher

$R^1$ die oben angegebene Bedeutung hat,

mit Aminoverbindungen der allgemeinen Formel (III)

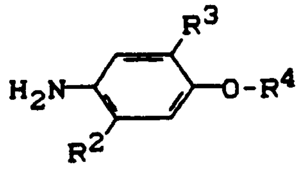

(III)

in welcher

$R^2$, $R^3$ und $R^4$ die oben angegebene Bedeutung haben,

gegebenenfalls in Gegenwart eines Verdünnungsmittels umsetzt.

Die neuen Verbindungen der allgemeinen Formel (I) verfügen über Eigenschaften, die ihre Verwendung als Schädlingsbekämpfungsmittel ermöglichen; sie zeigen gute insektizide Wirkung und zeichnen sich insbesondere durch sehr starke akarizide Wirksamkeit aus.

In den allgemeinen Formeln steht Fluor $R^1$ vorzugsweise in der 2- oder 4-Stellung, besonders bevorzugt in der 2-Stellung des Phenylringes.

$R^2$ steht in den allgemeinen Formeln vorzugsweise für Wasserstoff oder Chlor.

$R^3$ steht in den allgemeinen Formeln vorzugsweise für Wasserstoff oder Chlor.

Im Phenylring $R^4$, welcher durch Trifluormethyl substituiert ist, steht diese Gruppe in der 2-, 3- oder 4-Stellung, vorzugsweise in der 4-Stellung. Der zusätzlich durch Fluor und/oder Chlor substituierte Phenylring enthält 1 bis 4, vorzugsweise 1, 2 oder 3 Fluor- und/oder Chloratome, vorzugsweise Chloratome. Besonders bevorzugt werden hierbei Phenylringe, welche in der 2-Stellung oder in den 2-, 6-Stellungen oder in den 2-, 3-, 6-Stellungen durch Chlor substituiert vorliegen.

Chlortrifluorethyl $R^4$ bedeutet vorzugsweise $-CF_2-CHFCl$ und Hexafluorpropenyl bedeutet vorzugsweise $-C(CF_3)=CHCF_3$.

In den bevorzugten erfindungsgemäßen Verbindungen (bzw. den entsprechenden Ausgangsverbindungen) bedeutet $R^1$ Wasserstoff oder 2-Fluor, $R^2$ Wasserstoff oder Chlor, $R^3$ Wasserstoff und $R^4$ 4-Trifluormethyl (wobei $R^1$ für 2-Fluor steht), 4-Trifluormethyl-2,6-dichlorphenyl oder 4-Trifluormethyl-2,3,6-trichlorphenyl.

Bevorzugt werden die neuen Furazanylharnstoffe der Formel (I), in welcher

- $R^1$ für Wasserstoff oder Fluor steht,
- $R^2$ für Wasserstoff, Fluor oder Chlor steht,
- $R^3$ für Wasserstoff, Fluor oder Chlor steht und
- $R^4$ für Phenyl steht, welches in para-Position (4-Stellung) durch Trifluormethyl und zusätzlich einfach bis dreifach durch Fluor und/oder Chlor substituiert ist, und, für den Fall, daß $R^1$ für Fluor steht und/oder $R^2$ und/oder $R^3$ für Fluor oder Chlor stehen, $R^4$ darüber hinaus auch für Difluormethyl, Trifluormethyl, Chlordifluormethyl, Trifluorethyl, Tetrafluorethyl, Chlortrifluorethyl, Hexafluorpropyl oder Hexafluorpropenyl steht.

Für diese Restekombinationen gelten die oben aufgeführten Vorzugsdefinitionen jeweils entsprechend.

Verwendet man beispielsweise 3-Isocyanato-4-phenyl-1,2,5-oxadiazol und 3-Fluor-4-chlordifluormethoxy-anilin als Ausgangsstoffe, so kann der Reaktionsablauf beim erfindungsgemäßen Verfahren durch das folgende Formelschema wiedergegeben werden:

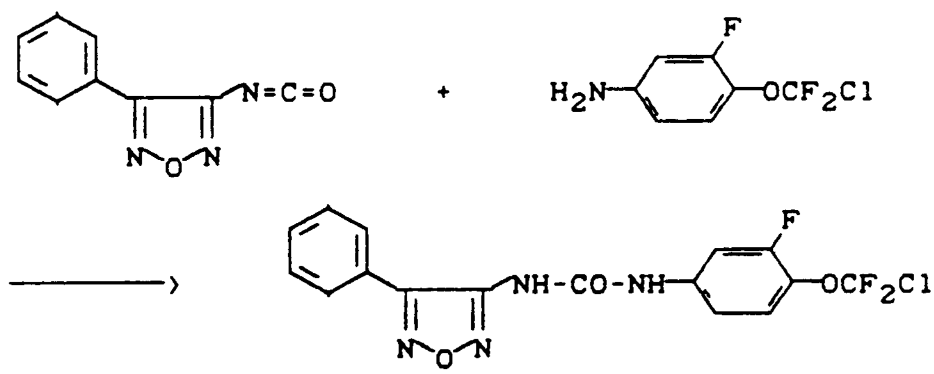

Die als Ausgangsstoffe zu verwendenden Furazanylisocyanate der Formel (II) sind bekannt und/oder können nach an sich bekannten Verfahren hergestellt werden (vgl. EP-A 0 132 680; US-P 4 699 916; US-P 4 826 988; EP-A 156 198).

Die weiter als Ausgangsstoffe zu verwendenden Aminoverbindungen der Formel (III) sind ebenfalls bekannt und/oder können nach an sich bekannten Verfahren hergestellt werden (vgl. DE-OS 2 411 320, EP-A 161 019, EP-A 216 423, DE-OS 3 603 089).

Neu und Teile der vorliegenden Erfindung sind die Aminoverbindungen der Formel (III), in welcher

- $R^2$ für Fluor oder Chlor (vorzugsweise Chlor) steht,
- $R^3$ für Wasserstoff, Fluor oder Chlor steht und
- $R^4$ für Phenyl steht, welches in para-Position (4-Stellung) durch Trifluormethyl und zusätzlich zweifach oder dreifach (vorzugsweise in 2,6-Stellung oder in 2,3,6-Stellung) durch Fluor und/oder Chlor (vorzugsweise durch Chlor) substituiert ist.

Man erhält die neuen Verbindungen der Formel (III), wenn man Aminophenole der allgemeinen Formel (IV)

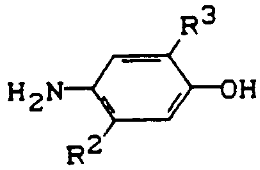

(IV)

in welcher

$R^2$ und $R^3$ die oben angegebene Bedeutung haben,

mit Halogenverbindungen der allgemeinen Formel (V)

$X\text{-}R^4$ (V)

in welcher

$R^4$ die oben angegebene Bedeutung hat und

X für Halogen, insbesondere für Fluor oder Chlor steht,

in Gegenwart eines Säureakzeptors, wie z.B. Kaliumcarbonat, und in Gegenwart eines Verdünnungsmittels, wie z.B. N-Methyl-pyrrolidon, bei Temperaturen zwischen 50 °C und 150 °C umsetzt und nach üblichen Methoden aufarbeitet (vgl. die Herstellungsbeispiele).

Das erfindungsgemäße Verfahren zur Herstellung der neuen Furazanylharnstoffe der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren in einem größeren Bereich variiert werden. Bevorzugt arbeitet man bei Temperaturen zwischen 0 °C und 200 °C, besonders bevorzugt bei Temperaturen zwischen 20 °C und 150 °C.

Das erfindungsgemäße Verfahren wird bevorzugt unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe vorzugsweise in angenähert äquimolaren Mengen eingesetzt. Es ist jedoch auch möglich, eine der beiden jeweils eingesetzten Komponenten in einem größeren Überschuß zu verwenden. Die Reaktionen werden vorzugsweise in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Verbindungen der Formel (I) zeichnen sich durch eine starke insektizide und besonders durch eine starke akarizide Wirksamkeit aus, insbesondere gegen Eier (ovizide Wirkung) und gegen die Larven (larvizide Wirkung) der Schädlinge.

Sie zeigen insbesondere beim Einsatz als Akarizide eine hervorragende Wirkung auf Eier und Larven von gemeiner Spinnmilbe (Tetranychus urticae) und Obstbaumspinnmilbe (Panonychus ulmi) und sind praktisch gegen alle Entwicklungsstadien von Spinnmilben sehr gut wirksam.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als

flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichteprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Bevorzugte erfindungsgemäße Schädlingsbekämpfungsmittel enthalten außer den erfindungsgemäßen Wirkstoffen gegebenenfalls neben üblichen Hilfs- und Trägerstoffen, wenigstens einen oberflächenaktiven Stoff (vorzugsweise einen als Emulgator und/oder als Netzmittel wirksamen Stoff).

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Alle Prozentangaben beziehen sich im vorliegenden Text auf Gewichtsprozente, wenn keine andere Angabe gemacht wird.

Die Herstellung der erfindungsgemäßen Verbindungen soll anhand der folgenden Beispiele erläutert werden.

Herstellungsbeispiele:

Beispiel 1

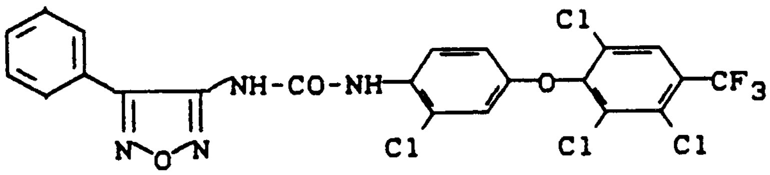

Eine Lösung von 1,5 g (8 mMol) 3-Isocyanato-4-phenyl-1,2,5-oxadiazol in 8 ml Toluol wird bei 20 °C unter Rühren zu einer Lösung von 3,13 g (8 mMol) 2-Chlor-4-(2,3,6-trichlor-4-trifluormethyl-phenoxy)-anilin in 50 ml Toluol tropfenweise gegeben. Das Reaktionsgemisch wird dann 60 Minuten bei 80 °C gerührt und wieder auf 20 °C abkühlen gelassen. Das kristallin angefallene Produkt wird durch Absaugen isoliert.

Man erhält 3,1 g (65% der Theorie) 1-[2-Chlor-4-(2,3,6-trichlor-4-trifluormethyl-phenoxy)-phenyl]-3-(4-phenyl-1,2,5-oxadiazol-3-yl)-harnstoff vom Schmelzpunkt 209 °C.

Analog Beispiel 1 und gemäß der allgemeinen Beschreibung des erfindungsgemäßen Herstellungsverfahrens können beispielsweise auch die in der nachstehenden Tabelle 1 aufgeführten Verbindungen der Formel (I) hergestellt werden.

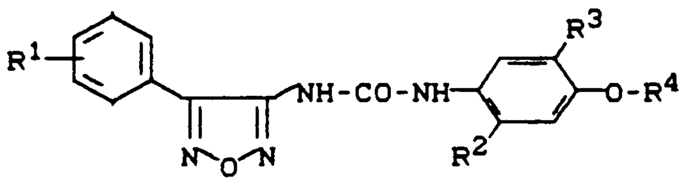

(I)

Tabelle 1: Beispiele für die Verbindungen der Formel (I)

| Bsp.-Nr. | (Position-) $R^1$ | $R^2$ | $R^3$ | $R^4$ | Schmelzpunkt (°C) |
|---|---|---|---|---|---|
| 2 | H | Cl | H | 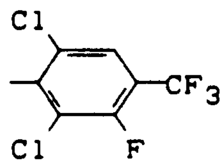 | 201 |
| 3 | (4-)F | H | H | 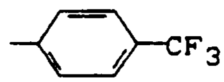 | |
| 4 | H | H | Cl | $CF_3$ | |
| 5 | H | H | Cl | $-CF_2CHFCl$ | |
| 6 | H | H | Cl | $-CF_2CHF_2$ | |
| 7 | (4-)F | H | H | $-CF_2CHFCl$ | |
| 8 | (4-)F | H | H | $-CF_2CHF_2$ | |
| 9 | H | H | Cl | $-CF_2CHFCl$ | 214 |

<u>Tabelle 1</u> - Fortsetzung

| Bsp.-Nr. | (Position-) $R^1$ | $R^2$ | $R^3$ | $R^4$ | Schmelzpunkt (°C) |
|---|---|---|---|---|---|
| 10 | H | Cl | H | $-CF_2CHFCl$ | 170 |
| 11 | (4-)F | H | Cl | $-CF_2CHF_2$ | 209 |
| 12 | (4-)F | H | Cl | $-CF_2CHFCl$ | 202 |
| 13 | (4-)F | H | H | $CF_3$ | 193 |
| 14 | (4-)F | H | H | $CHF_2$ | 190 |
| 15 | (4-)F | H | H | 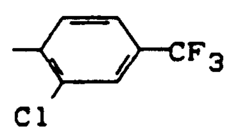 | 209 |
| 16 | (4-)F | H | H | 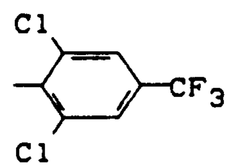 | 239 |
| 17 | H | Cl | H | 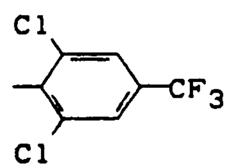 | 211 |
| 18 | (4-)F | Cl | H | 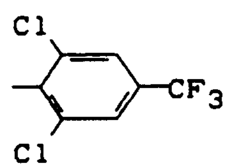 | 212 |
| 19 | (2-)F | H | H | $-CF_2CHFCl$ | 216 |
| 20 | (2-)F | H | H | $CF_3$ | 204 |
| 21 | (2-)F | H | H | $-CF_2CHF_2$ | 206 |
| 22 | (2-)F | H | Cl | $-CF_2CHFCl$ | 199 |

Tabelle 1 - Fortsetzung

| Bsp.-Nr. | (Position-) $R^1$ | $R^2$ | $R^3$ | $R^4$ | Schmelzpunkt (°C) |
|---|---|---|---|---|---|
| 23 | H | F | H | $-CF_2CHFCF_3$ | 208 |
| 24 | (4-)F | F | H | $-CF_2CHFCF_3$ | 205 |
| 25 | (2-)F | H | Cl | $CF_3$ | 214 |
| 26 | (2-)F | H | H | $CF_2Cl$ | 204 |
| 27 | (2-)F | H | H | $-CF_2CHFCF_3$ | 210 |
| 28 | (2-)F | F | H | $-CF_2CHFCF_3$ | |
| 29 | (2-)F | H | H | $CHF_2$ | 201 |
| 30 | (2-)F | Cl | H | $-C(CF_3)=CHCF_3$ | 196 |
| 31 | H | H | Cl | $-CH_2CF_3$ | 208 |
| 32 | (4-)F | H | H | $-CH_2CF_3$ | 210 |
| 33 | (2-)F | H | H | $-CH_2CF_3$ | 209 |
| 34 | (4-)F | H | Cl | $-CH_2CF_3$ | 196 |
| 35 | (2-)F | H | Cl | $-CH_2CF_3$ | 196 |
| 36 | H | F | H | $CF_3$ | |
| 37 | (4-)F | F | H | $CF_3$ | |
| 38 | (2-)F | F | H | $CF_3$ | |
| 39 | H | F | H | $CF_2Cl$ | |
| 40 | (4-)F | F | H | $CF_2Cl$ | |

<u>Tabelle 1</u> - Fortsetzung

| Bsp.-Nr. | (Position-) $R^1$ | $R^2$ | $R^3$ | $R^4$ | Schmelzpunkt (°C) |
|---|---|---|---|---|---|
| 41 | (2-)F | F | H | $CF_2Cl$ | |
| 42 | H | F | H | 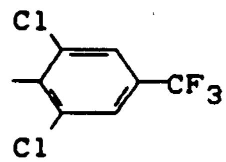 | |
| 43 | (4-)F | F | H | 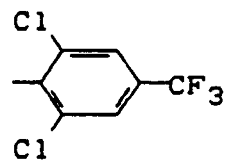 | |
| 44 | (2-)F | F | H | 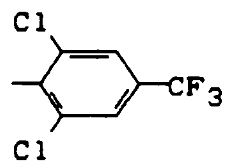 | |

<u>Ausgangsstoffe der Formel (III):</u>

<u>Beispiel (III-1)</u>

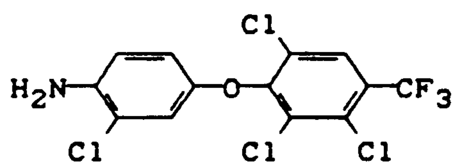

Eine Mischung aus 7,5 g (0,05 Mol) 3-Chlor-4-amino-phenol, 14,3 g (0,05 Mol) 2,3,4,5-Tetrachlorbenzotrifluorid und 6,9 g (0,05 Mol) Kaliumcarbonat in 35 ml N-Methyl-pyrrolidon wird 4 Stunden bei 125 °C bis 130 °C gerührt, dann unter Rühren auf etwa das doppelte Volumen Wasser gegeben und filtriert. Das feste Rohprodukt wird in heißem Diisopropylether aufgenommen und filtriert. Das Filtrat wird etwa zur Hälfte des Volumens eingeengt, das Produkt kristallisiert aus und wird durch Absaugen isoliert.

Man erhält 19 g (97% der Theorie) 2-Chlor-4-(2,3,6-trichlor-4-trifluormethyl-phenoxy)-anilin vom Schmelzpunkt 128 °C.

Analog Beispiel (III-1) können beispielsweise auch die in der nachstehenden Tabelle 2 aufgeführten neuen Verbindungen der Formel (III) hergestellt werden.

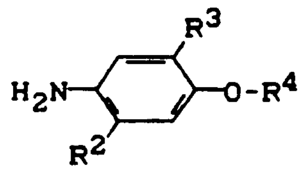

(III)

<u>Tabelle 2:</u> Beispiele für die Verbindungen der Formel (IIIa)

| Bsp.-Nr. | $R^2$ | $R^3$ | $R^4$ | Physikal. Daten |
|---|---|---|---|---|
| III-2 | Cl | H | 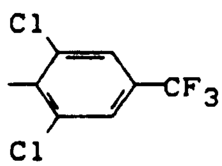 | |
| III-3 | Cl | F | 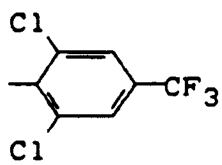 | |
| III-4 | Cl | H | 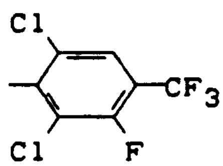 | |
| III-5 | Cl | F | 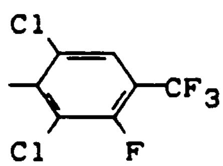 | |

<u>Tabelle 2</u> - Fortsetzung

| Bsp.-Nr. | $R^2$ | $R^3$ | $R^4$ | Physikal. Daten |
|---|---|---|---|---|
| III-6 | Cl | H | 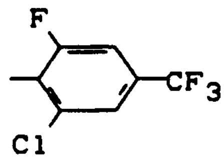 | |
| III-7 | Cl | H | 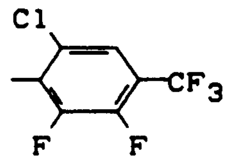 | |
| III-8 | Cl | F | 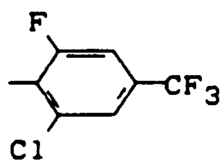 | |
| III-9 | Cl | F | 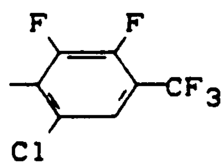 | |
| III-10 | Cl | H | 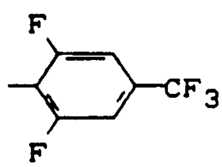 | |
| III-11 | Cl | H | 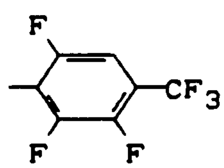 | |
| III-12 | Cl | F | 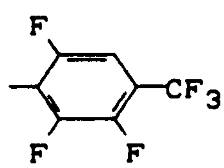 | |

Tabelle 2 - Fortsetzung

| Bsp.-Nr. | $R^2$ | $R^3$ | $R^4$ | Physikal. Daten |
|---|---|---|---|---|
| III-13 | Cl | H | 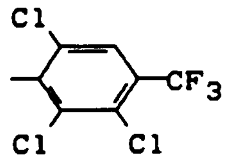 | |
| III-14 | Cl | F | 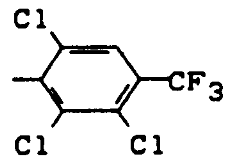 | |

Die biologische Wirksamkeit der erfindungsgemäßen Verbindungen soll anhand der folgenden Beispiele erläutert werden:

Anwendungsbeispiele:

Beispiel A

**Tetranychus-Test** (OP-resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 100%, daß alle Spinnmilben abgetötet wurden; 0% bedeutet, daß keine Spinnmilben abgetötet wurden.

Bei diesem Test zeigten zum Beispiel die Verbindungen der Herstellungsbeispiele (2) und (17) bei einer Konzentration von 0,000032% und die Verbindung des Herstellungsbeispiels (20) bei einer Konzentration von 0,00016% eine mindestens 90%ige Abtötung der Spinnmilben nach 14 Tagen.

**Patentansprüche**

**1.** Furazanylharnstoffe der allgemeinen Formel (I)

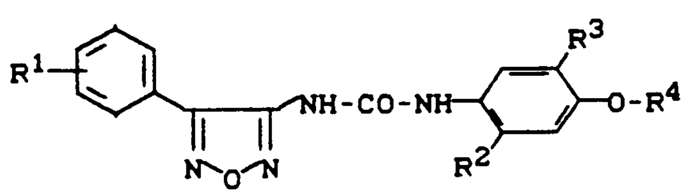

(I)

in welcher

$R^1$ für Wasserstoff oder Fluor steht,

$R^2$ für Wasserstoff, Fluor oder Chlor steht,

$R^3$ für Wasserstoff, Fluor oder Chlor steht und

$R^4$ für Phenyl steht, welches durch Trifluormethyl und zusätzlich einfach bis vierfach durch Fluor und/ oder Chlor substituiert ist, und, für den Fall, daß $R^1$ für Fluor steht und/oder $R^2$ und/oder $R^3$ für Fluor oder Chlor stehen, $R^4$ darüber hinaus auch für Difluormethyl, Trifluormethyl, Chlordifluormethyl, Trifluorethyl, Tetrafluorethyl, Chlortrifluorethyl, Hexafluorpropyl, Hexafluorpropenyl oder Trifluormethylphenyl steht.

**2.** Furazanylharnstoffe gemäß Anspruch 1, in welchen

$R^1$ für Wasserstoff oder Fluor steht,

$R^2$ für Wasserstoff, Fluor oder Chlor steht,

$R^3$ für Wasserstoff, Fluor oder Chlor steht und

$R^4$ für Phenyl steht, welches in para-Position (4-Stellung) durch Trifluormethyl und zusätzlich einfach bis dreifach durch Fluor und/oder Chlor substituiert ist, und,
für den Fall, daß $R^1$ für Fluor steht und/oder $R^2$ und/oder $R^3$ für Fluor oder Chlor stehen, $R^4$ darüber hinaus auch für Difluormethyl, Trifluormethyl, Chlordifluormethyl, Trifluorethyl, Tetrafluorethyl, Chlortrifluorethyl, Hexafluorpropyl oder Hexafluorpropenyl steht.

**3.** Furazanylharnstoffe gemäß Anspruch 1 der Formeln

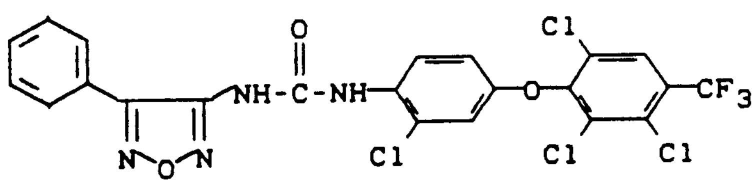

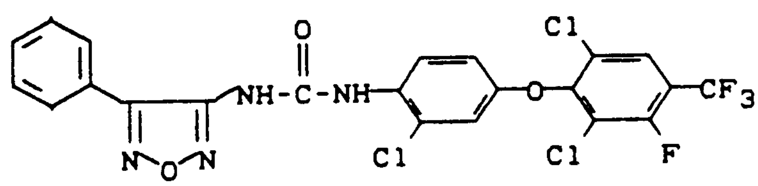

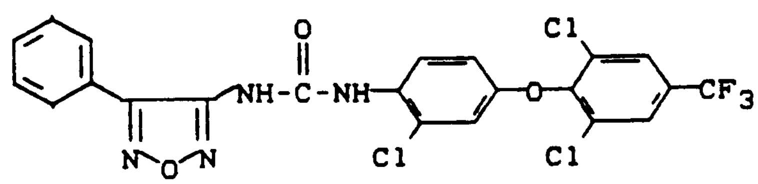

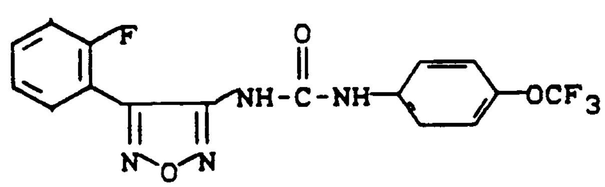

**4.** Verfahren zur Herstellung der Furazanylharnstoffe der allgemeinen Formel (I) gemäß Anspruch 1, dadurch gekennzeichnet, daß man Furazanylisocyanate der allgemeinen Formel (II)

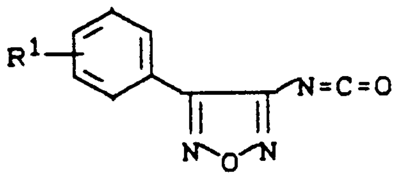

(II)

in welcher

$R^1$ die in Anspruch 1 angegebene Bedeutung hat,

mit Aminoverbindungen der allgemeinen Formel (III)

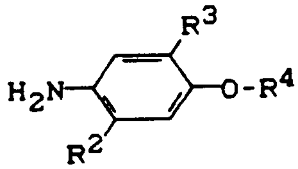

(III)

in welcher

$R^2$, $R^3$ und $R^4$ die in Anspruch 1 angegebene Bedeutung haben,

gegebenenfalls in Gegenwart eines Verdünnungsmittels umsetzt.

5. Schädlingsbekämpfungsmittel, gekennzeichnet durch einen Gehalt an mindestens einer Verbindung der Formel (I), gemäß Anspruch 1.

6. Verwendung von Verbindungen der Formel (I) gemäß Anspruch 1 zur Bekämpfung von Schädlingen.

7. Verfahren zur Bekämpfung von Schädlingen, dadurch gekennzeichnet, daß man Verbindungen der Formel (I) gemäß Anspruch 1 auf Schädlinge und/oder ihren Lebensraum einwirken läßt.

8. Verfahren zur Herstellung von Schädlingsbekämpfungsmitteln, dadurch gekennzeichnet, daß man Verbindungen der Formel (I) gemäß Anspruch 1 mit Streckmitteln und/oder oberflächenaktiven Mitteln vermischt.

9. Aminoverbindungen der allgemeinen Formel (III)

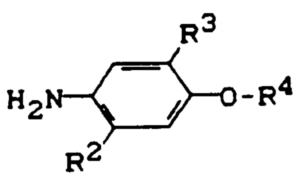

(III)

in welcher

$R^2$ für Fluor oder Chlor steht,

$R^3$ für Wasserstoff, Fluor oder Chlor steht und

$R^4$ für Phenyl steht, welches in para-Position (4-Stellung) durch Trifluormethyl und zusätzlich zweifach oder dreifach durch Fluor und/oder Chlor substituiert ist.

## Claims

**1.** Furazanylureas of the general formula (I)

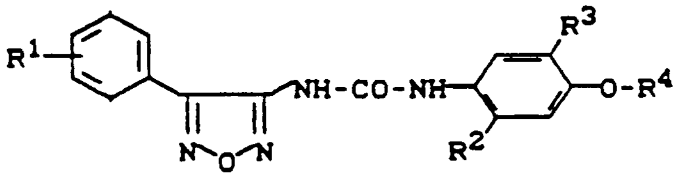

(I)

in which

$R^1$ represents hydrogen or fluorine,

$R^2$ represents hydrogen, fluorine or chlorine,

$R^3$ represents hydrogen, fluorine or chlorine and

$R^4$ represents phenyl which is substituted by trifluoromethyl and additionally monosubstituted to tetrasubstituted by fluorine and/or chlorine, and,

in the event that $R^1$ represents fluorine and/or $R^2$ and/or $R^3$ represent fluorine or chlorine, $R^4$ furthermore also represents difluoromethyl, trifluoromethyl, chlorodifluoromethyl, trifluoroethyl, tetrafluoroethyl, chlorotrifluoroethyl, hexafluoropropyl, hexafluoropropenyl or trifluoromethylphenyl.

**2.** Furazanylureas according to Claim 1, in which

$R^1$ represents hydrogen or fluorine,

$R^2$ represents hydrogen, fluorine or chlorine,

$R^3$ represents hydrogen, fluorine or chlorine and

$R^4$ represents phenyl which is substituted in the para-position (4-position) by trifluoromethyl and additionally monosubstituted to trisubstituted by fluorine and/or chlorine, and,

in the event that $R^1$ represents fluorine and/or $R^2$ and/or $R^3$ represent fluorine or chlorine, $R^4$ additionally also represents difluoromethyl, trifluoromethyl, chlorodifluoromethyl, trifluoroethyl, tetrafluoroethyl, chlorotrifluoroethyl, hexafluoropropyl or hexafluoropropenyl.

**3.** Furazanylureas according to Claim 1, of the formulae

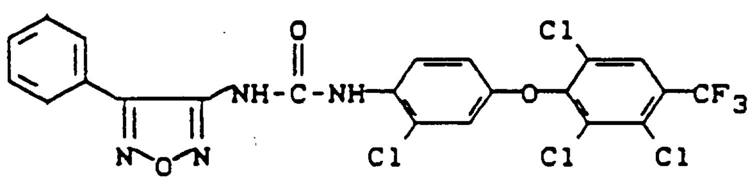

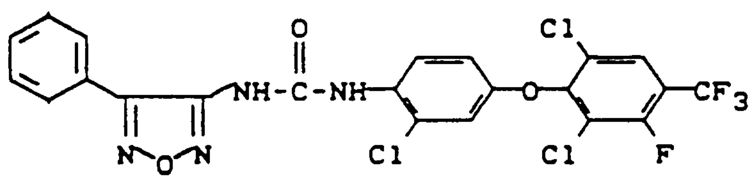

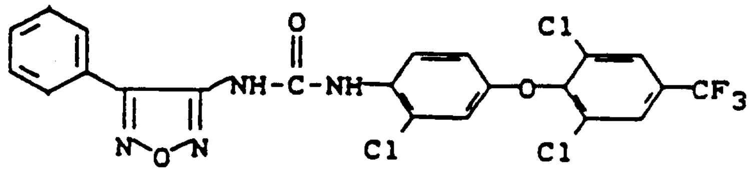

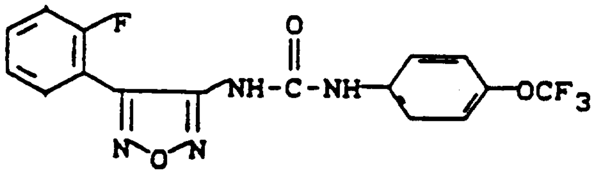

4. Process for the preparation of the furazanylureas of the general formula (I) according to Claim 1, characterized in that furazanyl isocyanates of the general formula (II)

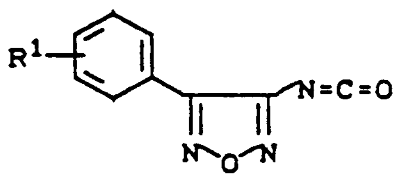

(II)

in which

$R^1$ has the meaning given in Claim 1,

are reacted with amino compounds of the general formula (III)

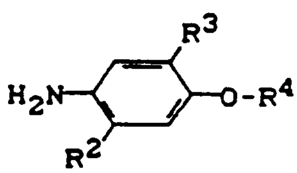

(III)

in which

$R^2$, $R^3$ and $R^4$ have the meaning given in Claim 1,

if appropriate in the presence of a diluent.

5. Pesticides, characterized in that they contain at least one compound of the formula (I) according to Claim 1.

6. Use of compounds of the formula (I) according to Claim 1 for combating pests.

7. Method of combating pests, characterized in that compounds of the formula (I) according to Claim 1 are allowed to act on pests and/or their environment.

8. Process for the preparation of pesticides, characterized in that compounds of the formula (I) according to Claim 1 are mixed with extenders and/or surface-active agents.

**9.** Amino compounds of the general formula (III)

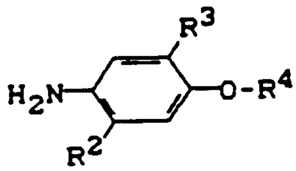

(III)

in which

$R^2$ represents fluorine or chlorine,

$R^3$ represents hydrogen, fluorine or chlorine and

$R^4$ represents phenyl which is substituted in the para-position (4-position) by trifluoromethyl and additionally disubstituted or trisubstituted by fluorine and/or chlorine.

**Revendications**

**1.** Furazanylurées de formule générale (I)

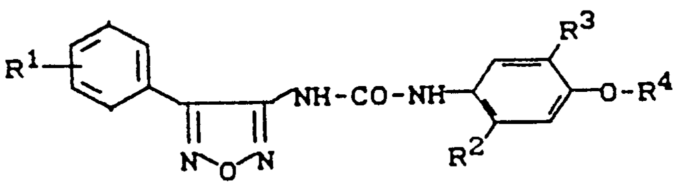

(I)

dans laquelle

$R^1$ représente de l'hydrogène ou du fluor,

$R^2$ représente de l'hydrogène, du fluor ou du chlore,

$R^3$ représente de l'hydrogène, du fluor ou du chlore et

$R^4$ est un groupe phényle qui est substitué par un radical trifluorométhyle et en outre une à quatre fois par du fluor et/ou du chlore et, au cas où $R^1$ représente du fluor et/ou $R^2$ et/ou $R^3$ représentent du fluor ou du chlore, $R^4$ représente en outre également un groupe difluorométhyle, trifluorométhyle, chlorodifluorométhyle, trifluoréthyle, tétrafluoréthyle, chlorotrifluoréthyle, hexafluoropropyle, hexafluoropropényle ou trifluorométhylphényle.

**2.** Furazanylurées suivant la revendication 1, dans lesquelles

$R^1$ représente de l'hydrogène ou du fluor,

$R^2$ représente de l'hydrogène, du fluor ou du chlore,

$R^3$ représente de l'hydrogène, du fluor ou du chlore et

$R^4$ est un groupe phényle qui est substitué en position para (position 4) par un radical trifluorométhyle et en outre une à trois fois par du fluor et/ou du chlore et, au cas où $R^1$ représente du fluor et/ou $R^2$ et/ou $R^3$ représentent du fluor ou du chlore, $R^4$ est en outre également un groupe difluorométhyle, trifluorométhyle, chlorodifluorométhyle, trifluoréthyle, tétrafluoréthyle, chlorotrifluoréthyle, hexafluoropropyle ou hexafluoropropényle.

3. Furazanylurées suivant la revendication 1, de formules

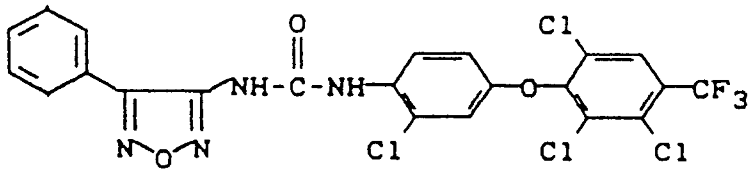

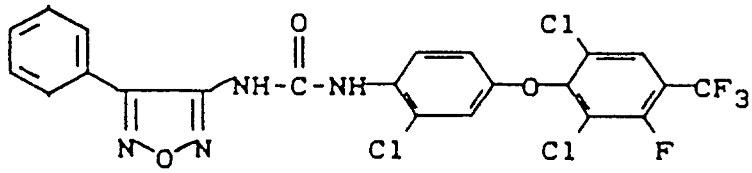

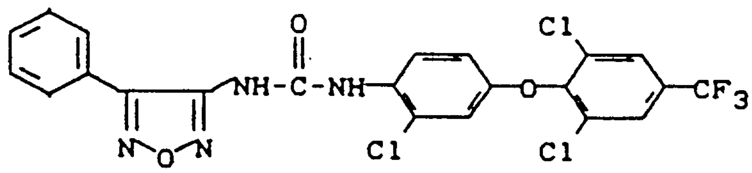

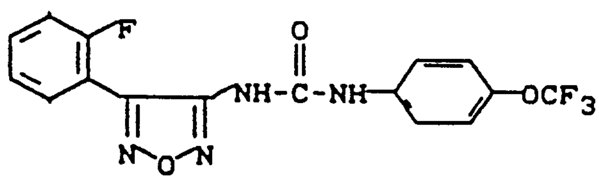

4. Procédé de production des furazanylurées de formule générale (I) suivant la revendication 1, caractérisé en ce qu'on fait réagir des furazanylisocyanates de formule générale (II)

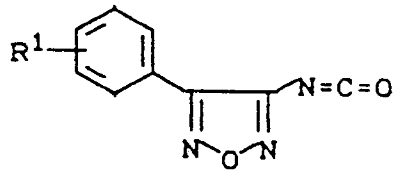

(II)

dans laquelle

$R^1$ a la définition indiquée dans la revendication 1,

avec des composés aminés de formule générale (III)

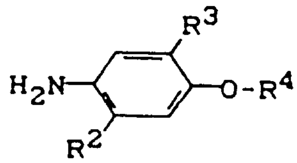

(III)

dans laquelle

$R^2$, $R^3$ et $R^4$ ont la définition indiquée dans la revendication 1,

le cas échéant en présence d'un diluant.

**5.** Compositions pesticides, caractérisées par une teneur en au moins un composé de formule (I) suivant la revendication 1.

**6.** Utilisation de composés de formule (I) suivant la revendication 1 pour combattre des parasites.

**7.** Procédé pour combattre des parasites, caractérisé en ce qu'on fait agir des composés de formule (I) suivant la revendication 1 sur les parasites et/ou sur leur milieu.

**8.** Procédé de préparation de compositions pesticides, caractérisé en ce qu'on mélange des composés de formule (I) suivant la revendication 1 avec des diluants et/ou des agents tensio-actifs.

**9.** Composés aminés de formule générale (III)

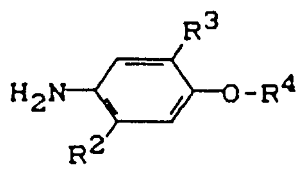

(III)

dans laquelle

$R^2$ représente du fluor ou du chlore,

$R^3$ représente de l'hydrogène, du fluor ou du chlore et

$R^4$ est un groupe phényle qui est substitué en position para (position 4) par un radical trifluorométhyle, et en outre deux ou trois fois par du fluor et/ou du chlore.